# EUROPÄISCHE PATENTANMELDUNG

(11) **EP 4 647 087 A2**
(43) Veröffentlichungstag der Anmeldung: **12.11.2025**
(21) Anmeldenummer: 25174780.4
(22) Anmeldetag: 07.05.2025
(51) Int. Cl.: A61L 2/22, B29C 49/42, B65B 55/10

(54) **VORRICHTUNG UND VERFAHREN ZUM BEHANDELN UND INSBESONDERE ZUM STERILISIEREN VON KUNSTSTOFFVORFORMLINGEN ODER BEHÄLTNISSEN**

(30) Priorität: 07.05.2024 DE 102024112824
(71) Anmelder: Krones AG, 93073 Neutraubling (DE)
(72) Erfinder: Söllner, Jürgen, 93073 Neutraubling (DE); Geltinger, Florian, 93073 Neutraubling (DE); Brikmann, Max, 93073 Neutraubling (DE)
(74) Vertreter: Bittner, Bernhard

(57) **Zusammenfassung**

Vorrichtung zum Behandeln und insbesondere Sterilisieren von Kunststoffvorformlingen und/oder Behältnissen mit einer Transporteinrichtung (2), welche dazu geeignet und bestimmt ist, die Kunststoffvorformlinge während ihrer Behandlung zu transportieren, mit einem einen Behandlungsraum (4a) ausbildenden Gehäuse (4), innerhalb dessen die Transporteinrichtung (2) angeordnet ist, mit einer Erzeugungseinrichtung (6) zum Erzeugen eines fließfähigen Behandlungsmittels und insbesondere Sterilisationsmittels und mit einer Zuführeinrichtung (8) welche dazu geeignet und bestimmt ist das von der Erzeugungseinrichtung erzeugte Sterilisationsmittel dem Behandlungsraum zuzuführen, dadurch gekennzeichnet, dass die Vorrichtung eine erste Abführeinrichtung (12) aufweist, welche dazu geeignet und bestimmt ist, aus dem Behandlungsraum ein mit dem Sterilisationsmittel angereichertes fließfähiges Medium zu entnehmen sowie eine Rückführungseinrichtung (22, 24, 26), welche dazu geeignet und bestimmt ist, wenigstens einen Teil des aus dem Behandlungsraum entnommenen Mediums wieder in diesen Behandlungsraum zurückzuführen.

## Beschreibung

Die vorliegende Erfindung bezieht sich auf eine Vorrichtung und ein Verfahren zum Behandeln und insbesondere zum Sterilisieren von Kunststoffvorformlingen oder Behältnissen. Im Stand der Technik ist es seit langem bekannt, beispielsweise Behältnisse oder Kunststoffvorformlinge vor bestimmten Behandlungsprozessen wie beispielsweise einem Füllprozess zu sterilisieren.

Im Falle von Kunststoffvorformlingen ist es in jüngerer Zeit bekannt geworden, diese zu sterilisieren, um sie dann mittels einer sterilen Blasformmaschine zu Kunststoffflaschen umzuformen. Zum Zwecke dieser Sterilisierung sind unterschiedliche Vorgehensweisen bekannt. So ist es beispielsweise bekannt, Behältnisse und/oder Kunststoffvorformlinge mittels UV-Strahlung oder mittels Röntgenstrahlung oder mittels Elektronenstrahlung zu sterilisieren.

Es ist jedoch im Stand der Technik auch seit langem bekannt, eine Sterilisation mittels fließfähigen Sterilisationsmitteln wie beispielsweise Wasserstoffperoxid oder Peressigsäure durchzuführen. Zu diesem Zweck werden üblicherweise die Kunststoffvorformlinge oder Behältnisse innerhalb eines Behandlungsraums mit einem Sterilisationsmittel fließfähigen beaufschlagt.

Hierbei tritt teilweise das Problem auf, dass der Verbrauch an Sterilisationsmitteln vergleichsweise hoch ist.

Der vorliegenden Erfindung liegt, daher die Aufgabe zugrunde, den Verbrauch an Sterilisationsmitteln zu reduzieren. Dies wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Behandeln und insbesondere Reinigen und/oder Sterilisieren von Kunststoffvorformlingen und/oder Behältnissen weist eine Transporteinrichtung auf, welche dazu geeignet und bestimmt ist, die Kunststoffvorformlinge und/oder Behältnisse während ihrer Behandlung wenigstens zeitweise zu transportieren. Bevorzugt werden diese Kunststoffvorformlinge (und/oder Behältnisse) wenigstens zeitweise und bevorzugt permanent während ihrer Behandlung transportiert. Bevorzugt werden diese Kunststoffvorformlinge (und/oder Behältnisse) kontinuierlich transportiert bzw. die Transporteinrichtung ist dazu geeignet und bestimmt, die Kunststoffvorformlinge (und/oder Behältnisse) während ihrer Behandlung kontinuierlich zu transportieren.

Weiterhin weist die Vorrichtung ein einen Behandlungsraum ausbildendes Gehäuse auf, innerhalb dessen die Transporteinrichtung angeordnet ist. Besonders bevorzugt umgibt dieses Gehäuse den Behandlungsraum im Wesentlichen vollständig (mit Ausnahme von etwaigen Schleusen, über welche Kunststoffvorformlinge und/oder Behältnisse in den Behandlungsraum zugeführt werden können oder von dem Behandlungsraum abgeführt werden können). Besonders bevorzugt schließt das Gehäuse den Behandlungsraum im Wesentlichen gasdicht ab (wiederum mit Ausnahme etwaiger Schleusen, welche der Zuführung oder Abführung von Kunststoffvorformlingen oder Behältnissen dienen).

Weiterhin weist die Vorrichtung eine Erzeugungseinrichtung zum Erzeugen eines fließfähigen Behandlungsmittels und/oder einen Vorrat zum Aufbewahren eines fließfähigen Behandlungsmittels auf. Insbesondere handelt es sich bei diesem Behandlungsmittel um ein Sterilisationsmittel.

Weiterhin ist eine Zuführeinrichtung vorgesehen, welche dazu geeignet und bestimmt ist, dass von der Erzeugungsmittel erzeugte Behandlungsmittel und insbesondere Sterilisationsmittel und/oder das in dem Aufbewahrungsmittel aufbewahrte Sterilisationsmittel dem Behandlungsraum zuzuführen.

Erfindungsgemäß weist die Vorrichtung eine erste Abführeinrichtung auf, welche dazu geeignet und bestimmt ist, aus dem Behandlungsraum ein fließfähiges Medium und insbesondere ein mit einem Sterilisationsmittel angereichertes fließfähiges Medium zu entnehmen sowie eine Rückführungseinrichtung, welche dazu geeignet und bestimmt ist, wenigstens einen Teil des aus dem Behandlungsraum entnommenen Mediums bzw. des fließfähigen Mediums wieder in den Behandlungsraum und/oder einen weiteren Behandlungsraum zurückzuführen.

Bevorzugt wird das fließfähige Medium aus einem ersten Behandlungsraum entnommen und über eine Rückführungseinrichtung einem zweiten Behandlungsraum zugeführt wird, wobei der erste Behandlungsraum ein anderer als der erste Behandlungsraum ist. Die Behandlungsräume können dem gleichen Typ entsprechen und/oder unterschiedlichen Typen, wie beispielsweise Füller, Verschließer, usw, bevorzugt Füller-, Verschließer-Behandlungsräume.

Bevorzugt wird das fließfähige Medium aus einem beliebigen, insbesondere dem ersten, Behandlungsraum entnommen und über eine Rückführungseinrichtung einer beliebigen Anzahl an weiteren Behandlungsräumen, bevorzugt jeweils einzeln, bevorzugt jedem weiteren Behandlungsraum in Teilen, zugeführt.

Wie erwähnt, tritt im Stand der Technik das Problem auf, dass eine effiziente Entkeimung eine hohe Strömungsgeschwindigkeit erfordert und damit eine hohe Menge an H₂O₂. Im Stand der Technik wird diese Menge vollständig aus einem Verdampfer oder der erwähnten Erzeugungseinrichtung bereitgestellt, was zu einem hohen Verbrauch an H₂O₂ Gas führt.

Im Rahmen der Erfindung wird daher vorgeschlagen, das aus der Behandlung der Kunststoffvorformlinge bzw. dem Behandlungsraum stammende Gas zu entnehmen und einem Frischgasstrom, insbesondere über einen Verdichter zuzuführen und/oder zuzumischen. Bevorzugt wird dabei, wie unten genauer beschrieben ein Steuern der Zirkulationsmenge über Druckmesser, Durchflussmesser und/oder Frequenzumformer durchgeführt. Der Frequenzumformer kann dabei dazu dienen, die Drehzahl des Verdichters zu steuern und somit den Druck bzw. Volumenstrom auf einen vorgegebenen Wert anzupassen.

Auf diese Weise kann eine Kosteneinsparung für einen Maschinenbetreiber um bis zu 66 %. erreicht werden. Daneben kann ein geringerer Verbrauch an insbesondere problematischen und/oder gesundheitsschädlichen Chemikalien bewirkt werden. Daneben ist auch eine deutlich einfachere Abschottung des Behandlungsraums oder der Gasbeaufschlagung gegenüber der Umgebung möglich.

Besonders bevorzugt handelt es sich bei der Erzeugungseinrichtung um einen Verdampfer. Besonders bevorzugt handelt es sich bei dem Sterilisationsmittel um ein Wasserstoffperoxid (H₂O₂) Gas.

Bei einer weiteren bevorzugten Ausführungsform weist die Vorrichtung wenigstens eine Mischeinrichtung auf, um wenigstens einen Teil des aus dem Behandlungsraum entnommenen fließfähigen Mediums mit dem von der Erzeugungseinrichtung erzeugten fließfähigen Medium zu mischen. Bei dieser Mischeinrichtung kann es sich im einfachsten Fall um einen Mischraum handeln, dem die beiden Komponenten zuführbar sind.

Dabei kann insbesondere ein Mischungsverhältnis zwischen diesen beiden Medien eingestellt und/oder gesteuert werden.

Besonders bevorzugt ist diese Mischeinrichtung außerhalb des Behandlungsraums angeordnet. So ist es möglich, dass diese Mischeinrichtung eine Vielzahl von Ventilen aufweist, mittels denen das Mischungsverhältnis zwischen dem aus dem Behandlungsraum entnommenen Medium und dem von der Erzeugungseinrichtung (frisch) hergestellten Medium bestimmt werden kann.

So ist es beispielsweise möglich, dass zum Zwecke des Mischens zwei oder mehrere Durchflussleitungen zusammengeführt werden. Daneben kann die Mischeinrichtung ein Mischraum aufweisen, in welchem eine Mischung einerseits des entnommenen Mediums und andererseits des hergestellten bzw. erzeugten Mediums erreicht wird.

Bei einer weiteren vorteilhaften Ausführungsform weist die Vorrichtung eine Steuerungseinrichtung auf, welche dazu geeignet und bestimmt ist, ein Mischungsverhältnis des dem Behandlungsraum zuzuführenden Mediums zu steuern und/oder zu regeln.

Insbesondere ist es dabei möglich, die Anteile des von der Erzeugungseinrichtung einerseits erzeugten Mediums und des von dem Behandlungsraum entnommenen Mediums, andererseits zu steuern.

So kann beispielsweise die Mischungseinrichtung so gesteuert werden, dass sich das dem Behandlungsraum zuzuführende Sterilisationsmedium zu 40 % aus frisch erzeugtem Sterilisationsmedium und zu 60 % aus von dem Behandlungsraum entnommenen Medium zusammensetzt.

Um dieses Mischungsverhältnis zu steuern und/oder zu regeln sind mehrere Vorgehensweisen denkbar. So ist es denkbar, dass eine Pumpleistung, mit der das fließfähige Medium aus dem Behandlungsraum entnommen wird, gesteuert und/oder geregelt wird. Auch kann das Mischungsverhältnis mit einer Vielzahl von Ventilen geregelt werden, welche in den jeweiligen Zufuhrleitungen für das aus dem Behandlungsraum entnommene Medium und das von der Erzeugungseinrichtung erzeugte Medium angeordnet sind.

Daneben ist es möglich, in der Mischeinrichtung bzw. einem Mischbereich ein Mischventil vorzusehen, welches die besagten Anteile steuert oder regelt.

Bei einer weiteren vorteilhaften Ausführungsform weist die Vorrichtung eine zweite Abführeinrichtung auf, welche dazu geeignet und bestimmt ist, aus dem Behandlungsraum ein fließfähiges Medium und insbesondere ein mit dem Sterilisationsmittel angereichertes fließfähiges Medium zu entnehmen, wobei bevorzugt diese zweite Abführeinrichtung von der ersten Abführeinrichtung beanstandet ist.

So können beispielsweise die wenigstens zwei Abführeinrichtungen an einander gegenüberliegenden Wandungen des oben genannten Gehäuses angeordnet sein. Besonders bevorzugt befindet sich die Transporteinrichtung wenigstens teilweise zwischen den wenigstens zwei Abführeinrichtungen. Auf diese Weise ist es möglich, das fließfähige Medium aus mehreren Bereichen des Gehäuses oder des Behandlungsraums abzunehmen bzw. abzuführen, wodurch insbesondere eine gleichmäßige Abnahme bzw. Abführung möglich ist.

Bevorzugt ist auch eine Rückführungseinrichtung vorgesehen, welche dazu geeignet und bestimmt ist, wenigstens einen Teil des von der zweiten Abführeinrichtung aus dem Behandlungsraum entnommenen Mediums wieder in diesen Behandlungsraum zurückzuführen. Diese Rückführungseinrichtung ist bevorzugt dazu geeignet und bestimmt, das aus dem Behandlungsraum abgeführte Medium wieder dem Behandlungsraum zuzuführen, wobei bevorzugt diese Zuführung erfolgt, nachdem dieses Medium mit frisch erzeugten Sterilisationsmedium gemischt wurde.

Es ist dabei möglich, dass die Fließmittelströme der beiden Abführeinrichtungen zunächst zusammengeführt werden und dann mit dem Strom des frisch erzeugten Sterilisationsmittels zusammengeführt werden, es wäre jedoch auch denkbar, dass die Fließmittelströme der Abführeinrichtungen hintereinander dem Strom des frisch erzeigten Sterilisationsmittels zugeführt werden. Auch wäre es denkbar, dass die Fließmittelströme der beiden Abführeinrichtungen gemeinsam mit dem frisch erzeugten Sterilisationsmittel einem gemeinsamen Mischraum zugeführt werden.

Bei einer weiteren bevorzugten Ausführungsformen ist die Mischeinrichtung dazu geeignet und bestimmt, die von beiden Rückführungseinrichtungen entnommenen Medium mit dem frisch erzeugten Sterilisationsmedium zu mischen.

Bei einer weiteren vorteilhaften Ausführungsformen weist die Vorrichtung wenigstens eine innerhalb des Behandlungsraums angeordnete Beaufschlagungseinrichtung auf, um die von der Transporteinrichtung transportierten Kunststoffvorformlinge und/oder Behältnisse mit dem fließfähigen Medium und insbesondere mit dem fließfähigen Sterilisationsmittel zu beaufschlagen. Insbesondere ist diese Beaufschlagungseinrichtung dazu geeignet und bestimmt, die Kunststoffvorformlinge mit der bereits gemischten Substanz zu beaufschlagen.

Besonders bevorzugt weist die Beaufschlagungseinrichtung bewegliche Beaufschlagungselemente auf und insbesondere Beaufschlagungselemente, welche gemeinsam mit den zu sterilisierenden Kunststoffvorformlingen mitbewegt werden.

Besonders bevorzugt weist die Beaufschlagungseinrichtung ein Leitungssystem auf, welches bevorzugt eine Vielzahl von Behandlungsdüsen aufweist, welche die Kunststoffvorformlinge mit dem Sterilisationsmittel beaufschlagen.

Bei einer weiteren vorteilhaften Ausführungsformen ist auch eine Verteileinrichtung und insbesondere ein Drehverteiler vorgesehen, um das fließfähige Medium und insbesondere das Sterilisationsmittel (insbesondere innerhalb des Behandlungsraums) auf eine Vielzahl von auf einem Transportträger und/oder Sterilisationsrad angeordnete Beaufschlagungseinrichtungen und insbesondere Beaufschlagungsdüsen zu verteilen.

Bei einer weiteren bevorzugten Ausführungsformen weist die Transporteinrichtung wenigstens einen ersten um eine vorgegebene Drehachse drehbaren Transportträger auf, an dem eine Vielzahl von Halteeinrichtungen zum Halten der Kunststoffvorformlinge oder Behältnisse angeordnet ist. Bevorzugt sind diese Halteeinrichtungen äquidistant an dem drehbaren Transportträger angeordnet. Besonders bevorzugt sind diese Halteeinrichtungen derart angeordnet, dass die Kunststoffvorformlinge hintereinander transportiert werden. Bevorzugt sind die Halteeinrichtungen derart angeordnet, dass von diesen Halteeinrichtungen gehaltene aufeinanderfolgende Kunststoffvorformlinge zueinander einen Abstand aufweisen, der größer ist als 2cm, bevorzugt größer als 3cm und bevorzugt größer als 4cm.

Bevorzugt sind die Halteeinrichtungen derart angeordnet, dass von diesen Halteeinrichtungen gehaltene aufeinanderfolgende Kunststoffvorformlinge zueinander einen Abstand aufweisen der kleiner ist als 20cm, bevorzugt kleiner als 18cm, bevorzugt kleiner als 15cm, bevorzugt kleiner als 12cm und besonders bevorzugt kleiner als 10cm.

Besonders bevorzugt sind diese Halteeinrichtungen resistent gegen das Sterilisationsmittel. Besonders bevorzugt handelt es sich bei den Halteeinrichtungen um Greifeinrichtungen, welche dazu geeignet und bestimmt sind, die Kunststoffvorformlinge oder Behältnisse im Bereich ihrer Mündungen und insbesondere unterhalb eines Tragrings der Kunststoffvorformlinge oder Behältnisse zu greifen.

Bei einer weiteren bevorzugten Ausführungsformen ist eine Zuführeinrichtung vorgesehen, welche den Halteeinrichtungen der Transporteinrichtung die Kunststoffvorformlinge oder Behältnisse zuführt. Bevorzugt ist auch diese Zuführeinrichtung innerhalb des Behandlungsraums angeordnet. Bevorzugt weist auch diese Zuführeinrichtung Beaufschlagungseinrichtungen zum Beaufschlagen der Kunststoffvorformlinge oder Behältnisse mit dem fließfähigen Sterilisationsmittel auf. Bevorzugt sind innerhalb des Behandlungsraums wenigstens drei und bevorzugt genau drei mit Halteeinrichtungen bestückte Transporträder vorgesehen, und die Kunststoffvorformling werden jeweils von allen diesen Transporträdern transportiert oder sind nacheinander von allen diesen Transporträdern transportierbar.

Besonders bevorzugt weist die Vorrichtung auch eine Abführeinrichtung auf, welche dazu geeignet und bestimmt ist, die Kunststoffvorformlinge oder Behältnisse von der Transporteinrichtung abzuführen. Bevorzugt ist auch diese Abführeinrichtung innerhalb des Behandlungsraums angeordnet. Bevorzugt weist auch diese Abführeinrichtung Beaufschlagungseinrichtungen zum Beaufschlagen der Kunststoffvorformlinge oder Behältnisse mit dem fließfähigen Sterilisationsmittel auf. Bevorzugt ist auch diese Abführeinrichtung ein Bestandteil der Transporteinrichtung.

Bei einer weiteren bevorzugten Ausführungsformen weist auch die Zuführeinrichtung einen drehbaren Träger auf, an dem eine Vielzahl von Halteeinrichtungen angeordnet ist. Bei einer weiteren bevorzugten Ausführungsformen weist auch die Abführeinrichtung einen drehbaren Träger auf, an dem eine Vielzahl von Halteeinrichtungen angeordnet ist. Besonders bevorzugt ist eine Drehachse der Zuführeinrichtung und/oder der Abführeinrichtung parallel zu der Drehachse der Transporteinrichtung angeordnet.

Besonders bevorzugt sind daher wenigstens drei derartige drehbare Träger und bevorzugt genau drei derartige Träger innerhalb des Behandlungsraums angeordnet.

Bei einer weiteren bevorzugten Ausführungsformen weist die Vorrichtung wenigstens eine Sensoreinrichtung auf, welche dazu geeignet und bestimmt ist, wenigstens einen Messwert zu erfassen, der für das innerhalb des Behandlungsraum befindliche fließfähige Medium charakteristisch ist.

Besonders bevorzugt ist dieser Messwert aus einer Gruppe von Messwerten ausgewählt, welche Druckmesswerte, Temperaturmesswerte, Messwerte für einen Anteil eines Sterilisationsmittels, Messwerte für eine Konzentration des Sterilisationsmittels, Luftfeuchtigkeitsmesswerte und dergleichen enthält.

Besonders bevorzugt ist diese Sensoreinrichtung innerhalb des Behandlungsraums angeordnet. Bevorzugt sind mehrere Sensoreinrichtungen vorgesehen und insbesondere sind diese auch in unterschiedlichen Positionen innerhalb des Behandlungsraums angeordnet.

Bei einer weiteren vorteilhaften Ausführungsformen weist die Vorrichtung wenigstens eine Durchflussmesseinrichtung und besonders bevorzugt mehrere Durchflussmesseinrichtungen auf. Dabei kann beispielsweise ein Durchfluss des aus dem Behandlungsraum entnommenen Mediums oder auch ein Durchfluss des erzeugten Sterilisationsmittels gemessen werden.

Besonders bevorzugt weist die Vorrichtung eine Vielzahl von Durchflussleitungen auf, insbesondere eine Durchflussleitung zum Fördern des aus dem Behandlungsraum entnommenen Mediums und auch eine Durchflussleitung zum Fördern des (frisch von der Erzeugungseinrichtung) erzeugten Mediums. Besonders bevorzugt können auch in diesen Durchflussleitungen Durchflussmesseinrichtungen vorgesehen sein.

Bei einer weiteren vorteilhaften Ausführungsform weist die Vorrichtung wenigstens eine Verdichtereinrichtung zur Verdichtung des von der Erzeugungseinrichtung erzeugten fließfähigen Mediums auf. Bei einer weiteren vorteilhaften Ausführungsformen weist die Vorrichtung wenigstens eine Verdichtereinrichtung zur Verdichtung des aus dem Behandlungsraum entnommenen fließfähigen Mediums auf.

Bevorzugt handelt es sich bei wenigstens einer Verdichtereinrichtung um ein Gebläse oder um einen Seitenkanalverdichter. Durch die Verwendung dieser Verdichtereinrichtung kann, wie oben erwähnt Gas direkt aus dem Behandlungsraum entnommen werden und einen zugeführten (frischen) Gasstrom, d. h. dem erzeugten Medium beigemengt werden.

Auf diese Weise kann auch die Strömungsgeschwindigkeit an den Behandlungsorganen beispielsweise den oben erwähnten Halteeinrichtungen für die Kunststoffvorformlinge oder Behältnisse erhöht werden, ohne dass Frischgas oder neu erzeugtes Gas erforderlich wird.

Bei einer weiteren bevorzugten Ausführungsform weist die Vorrichtung eine Beaufschlagungseinrichtung auf, welche dazu geeignet und bestimmt ist, mit wenigstens einen Teil des in den Behandlungsraum zurückzuführenden Mediums wenigstens einen Bereich der Kunststoffvorformlinge und insbesondere eine Außenoberfläche der Kunststoffvorformlinge zu beaufschlagen.

Bei den oben beschriebenen Ausführungsformen wurde eine Innenbehandlung der Kunststoffvorformling dargestellt. Im Rahmen dieser Ausführungsform wird vorgeschlagen, das wieder in den Behandlungsraum zurückzuführende Medium auch für eine Behandlung der Außenflächen der Kunststoffvorformlinge und/oder Kunststoffbehältnisse zu verwenden.

Bevorzugt werden für diese Außenbehandlungen der Kunststoffvorformlinge und/oder Kunststoffbehältnisse Düsen verwendet, welche deren Außenoberflächen beaufschlagen.

Bei dieser Neuentwicklung der Außenbehandlung wurde der Ansatz verfolgt, die Behandlung der Kunststoffvorformlinge durch zusätzliche Düsen zu realisieren. Dabei wird das Gasgemisch (insbesondere ein Gemisch aus H2O2 und Luft) das sich bereits im Reinraum befindet angesaugt, bevorzugt komprimiert und/oder verdichtet und den Beaufschlagungseinrichtungen und insbesondere Düsen zugeführt.

Bevorzugt ist auch hier wieder eine Verdichtereinrichtung und/oder Komprimierungseinrichtung vorgesehen, welche das in den Behandlungsraum rückzuführende Gemisch verdichtet und/oder komprimiert.

Bevorzugt weist die Vorrichtung im Inneren des Behandlungsraums angeordnete Düsen auf, die derart ausgerichtet und/oder angeordnet sind, dass sie eine Außenoberfläche der Kunststoffbehältnisse (oder insbesondere) Kunststoffvorformlinge beaufschlagen.

Bevorzugt sind diese Beaufschlagungseinrichtungen wenigstens teilweise unterhalb der Kunststoffbehältnisse und/oder Kunststoffvorformlinge angeordnet und beaufschlagen diese vorteilhaft von unten her.

Bevorzugt sind diese Beaufschlagungseinrichtungen und/oder Düsen derart angeordnet, dass es zu einer gezielten Überströmung der Außenflächen kommt. Diese Düsen können dabei bereits vorhandene Düsen unterstützen.

Bevorzugt erfolgt ein Ansaugen des Mediums aus dem Behandlungsraum an einer unteren Seite des Behandlungsraums und/oder von unten her (d.h. in einer vertikalen Richtung von unterhalb her). Durch dieses Ansaugen an der unteren Seite wird bevorzugt die grundsätzliche Überströmung der vorhandenen Flächen verbessert.

Bevorzugt ist es in diesem Fall möglich, dass Ventilatoren, welche bei vergleichbaren Anlagen in einem Dach des Behandlungsraums angeordnet sind, entfallen können.

Bei einer weiteren bevorzugten Ausführungsform sind die Beaufschlagungseinrichtungen und/oder Düsen höhenverstellbar angeordnet. Dabei kann bevorzugt eine derartige Höhenverstellbarkeit stufenlos erfolgen. Dabei kann bevorzugt eine derartige Höhenverstellbarkeit automatisiert, bevorzugt stufenlos erfolgen, beispielsweise in Abhängigkeit von einer Länge der Kunststoffbehältnisse und/oder Kunststoffvorformlinge.

Bei einer weiteren bevorzugten Ausführungsform kann ein Gebläse vorgesehen sein, um die Außenoberflächen der Kunststoffvorformlinge oder Kunststoffbehältnisse zu beaufschlagen. Dabei kann weiterhin eine weitere Durchflussmesseinrichtung vorgesehen sein, um eine Steuerung dieses Gebläses zu ermöglichen.

Bei einer bevorzugten Ausführungsform ist das Gebläse derart ausgebildet, dass es mit dem Sterilisationsmedium beaufschlagbar ist. Insbesondere weist dieses Gebläse Komponenten aus H2O2 - beständigen Materialien auf.

Diese Vorgehensweise, insbesondere zur Außenbehandlung der Kunststoffvorformlinge bringt mehrere Vorteile mit sich. Zunächst muss für die Außenbehandlung der Kunststoffvorformlinge oder Kunststoffbehältnisse kein Frischgasgemisch zur Verfügung gestellt werden. Es erfolgt vielmehr eine Nutzung von vorhandenem ausgeströmten Gas in dem Behandlungsraum bzw. Reinraum.

Weiterhin kann durch die beschriebene Vorgehensweise eine Reduzierung der Temperatur und/oder der Konzentration von Frischgas ermöglicht werden. Daneben ist die hier vorgeschlagene Vorgehensweise bevorzugt auch als Nachrüstung in bestehende Anlagen umsetzbar.

Bevorzugt sind die Beaufschlagungseinrichtungen und/oder Düsen in einem durch den Rüstzugang einfach zugänglichen Bereich angeordnet. Das heißt eine einfache Zugänglichkeit an die Beaufschlagungseinrichtungen und/oder Düsen soll von dem Rüstzugang aus möglich sein, beziehungsweise erreichbar sein. Bevorzugt sind die Beaufschlagungseinrichtungen und/oder Düsen beabstandet, insbesondere ausreichend beabstandet, von Verbindungsstellen zu sonstigen, insbesondere benachbarten Modulen angeordnet. Das heißt, es soll ein ausreichender Abstand zwischen den Verbindungsstellen eingehalten werden, um die Module weiterhin eigenständig behandeln und/oder handhaben zu können. Beabstandet, insbesondere ausreichend beabstandet wird hier als ein für den Fachmann üblicher Abstand angesehen, der den Umgang mit den Modulen besonders zum Ein-/Ausbau, zur Wartung und Instandhaltung, sowie zur Handhabbarkeit, sicherstellt.

Bevorzugt sind die Beaufschlagungseinrichtungen und/oder Düsen sowohl in einem einfach zugänglichen Bereich durch den Rüstzugang als auch beabstandet von den Verbindungsstellen zu sonstigen Modulen angeordnet.

Die vorliegende Erfindung ist weiterhin auf ein Verfahren zum Behandeln und insbesondere Sterilisieren von Kunststoffvorformlingen und/oder Behältnissen (und insbesondere Kunststoffbehältnissen) gerichtet, mit einer Transporteinrichtung, welche die Kunststoffvorformlinge und/oder Behältnisse wenigstens zeitweise während ihrer Behandlung transportiert.

Weiterhin ist ein einen Behandlungsraum ausbildendes Gehäuse vorgesehen, innerhalb dessen die Transporteinrichtung angeordnet ist und eine Erzeugungseinrichtung, welche ein fließfähiges Behandlungsmittel und insbesondere ein fließfähiges Sterilisationsmittel erzeugt. Weiterhin ist auch eine Zuführeinrichtung vorgesehen, welche das von der Erzeugungseinrichtung erzeugte Sterilisationsmittel dem Behandlungsraum zuführt.

Besonders bevorzugt ist eine erste Abführeinrichtung vorgesehen, welche aus dem Behandlungsraum ein mit dem Sterilisationsmittel angereichertes fließfähiges Medium entnimmt und auch eine Rückführungseinrichtung, welche wenigstens einen Teil des aus dem Behandlungsraum entnommenen fließfähigem Mediums wieder diesem Behandlungsraum zuführt bzw. dieses in den Behandlungsraum zurückführt.

Bevorzugt weist die Vorrichtung eine Aufbereitungseinrichtung auf, welche dazu geeignet und bestimmt ist, das aus dem Behandlungsraum entnommene Medium aufzubereiten, insbesondere bevor es mit dem frisch erzeugten fließfähigen Medium gemischt wird. Denkbar ist diesbezüglich beispielsweise eine Erhitzungsvorrichtung zum Nachheizen des entnommenen Behandlungsgases.

Es wird daher auch verfahrensseitig vorgeschlagen, dass wenigstens ein Teil des Sterilisationsmediums wiederverwendet wird, bzw. aus dem Behandlungsraum entnommen und wieder in diesen zurückgeführt wird. Besonders bevorzugt wird jedoch zusätzlich erzeugtes Sterilisationsmittel dem Behandlungsraum zugeführt. Besonders bevorzugt werden die Mengen des rückgeführten Sterilisationsmediums und die neu erzeugten Mengen des Sterilisationsmediums bzw. deren Anteile gesteuert.

Besonders bevorzugt wird dem Behandlungsraum (frisch) erzeugtes Sterilisationsmedium in einer Menge zugeführt, die größer ist als 100 kg/h bevorzugt größer als 200 kg/h, bevorzugt größer als 300 kg/h und bevorzugt größer als 400 kg/h.

Besonders bevorzugt ist die dem Behandlungsraum zugeführte Menge an (frisch) erzeugtem Sterilisationsmedium, d. h. an neu erzeugten Sterilisationsmittel geringer als 1000 kg/h bevorzugt geringer als 800 kg/h, bevorzugt geringer als 700 kg/h bevorzugt geringer als 600 kg/h.

Besonders bevorzugt liegt die Menge des dem Behandlungsraum entnommenen und dem Behandlungsraum wieder zurückgeführten Sterilisationsmittels bei mehr als 200kg/h, bevorzugt bei mehr als 400 kg/h bevorzugt bei mehr als 600 kg/h und bevorzugt bei mehr als 800kg/h. Bevorzugt liegt die Menge des dem Behandlungsraum entnommenen und zurückgeführten Sterilisationsmittels und/oder fließfähigen Mediums unter 2000 kg/h bevorzugt unter 1800 kg/h bevorzugt unter 1600 kg/h und bevorzugt unter 1400 kg/h.

Besonders bevorzugt ist daher der Anteil des von dem Behandlungsraum entnommenen und in den Behandlungsraum zurückgeführten Sterilisationsmediums höher als der Anteil an neu erzeugtem Sterilisationsmediums.

Besonders bevorzugt werden die Kunststoffvorformlinge oder Behältnisse im Inneren des Behandlungsraum wenigstens abschnittsweise auf einem kreisförmigen Transportpfad transportiert. Besonders bevorzugt werden innerhalb des Behandlungsraums Kunststoffvorformlinge auf wenigstens zwei unterschiedlichen Transportebenen bewegt, welche sich insbesondere in der Richtung der Drehachse der Transporteinrichtung unterscheiden und besonders bevorzugt in Richtung der Drehachse übereinander angeordnet sind und senkrecht zu der Drehachse stehen. Auf diese Weise kann die Effizienz der Sterilisation innerhalb des Behandlungsraums erhöht werden.

Bei einem weiteren bevorzugten Verfahren werden die Kunststoffvorformlinge oder Behältnisse innerhalb des Behandlungsraums wenigstens einmal und bevorzugt wenigstens zweimal von einer ersten Halteeinrichtung an eine zweite Halteeinrichtung übergeben.

Bei einem weiteren bevorzugten Verfahren werden die Kunststoffvorformlinge und/oder (Kunststoff)behältnisse innerhalb des Behandlungsraums wenigstens einmal in unterschiedlichen Drehrichtungen transportiert. Besonders bevorzugt werden die Kunststoffvorformlinge oder Behältnisse mit dem Sterilisationsmittel beaufschlagt.

Besonders bevorzugt wird auch eine Innenoberfläche der Kunststoffvorformlinge oder Behältnisse mit dem Sterilisationsmittel beaufschlagt. Dies erfolgt bevorzugt mittels Düsen, welche dazu geeignet sind, Sterilisationsmittel in den Innenraum der Kunststoffvorformlinge einzubringen.

Bei einem weiteren bevorzugten Verfahren wird das Sterilisationsmittel mittels eines Dreh Verteilers auf bewegliche und insbesondere auf einem drehbaren Träger und insbesondere dem oben erwähnten Transportträger befindliche Düsen verteilt.

Besonders bevorzugt ist jeder der oben genannten Halteeinrichtungen genau eine und/oder wenigstens eine Beaufschlagungseinrichtung zugeordnet.

Bei einem weiteren bevorzugten Verfahren wird in einer Mischeinrichtung ein Teil des aus dem Behandlungsraum entnommenen fließfähigen Mediums mit dem von der Erzeugungseinrichtung erzeugten fließfähigen Medium gemischt. Besonders bevorzugt erfolgt dabei dieses Mischen außerhalb des Behandlungsraums.

Bei einem weiteren bevorzugten Verfahren wird ein Mischungsverhältnis zwischen dem von der Erzeugungseinrichtung erzeugten Medium und dem von dem Behandlungsraum entnommenen Medium gesteuert und/oder geregelt. Dies kann beispielsweise mittels einer Pumpeneinrichtung, welche die Förderleistung ändern kann, oder mittels Ventilen erfolgen.

Bei einem weiteren bevorzugten Verfahren werden die Kunststoffvorformlinge und/oder Behältnisse dem Behandlungsraum mittels einer Schleuseneinrichtung zugeführt.

Bei einem weiteren bevorzugten Verfahren werden die Kunststoffvorformlinge und/oder Behältnisse aus dem Behandlungsraum mittels einer Schleuseneinrichtung entnommen oder aus diesem abgeführt. Bevorzugt reduziert diese Schleuseneinrichtung den Austritt von fließfähigem Medium aus dem Behandlungsraum.

Bei einem weiteren bevorzugten Verfahren werden die Kunststoffvorformlinge erwärmt, bevor sie dem Behandlungsraum zugeführt werden.

Besonders bevorzugt werden die Kunststoffvorformlinge nach ihrer Sterilisation mittels einer Umformungseinrichtung zum Umformen von Kunststoffvorformlingen zu Kunststoffbehältnissen umgeformt. Besonders bevorzugt handelt es sich hierbei um eine sterile Umformungseinrichtung, insbesondere um eine sterile Blasformmaschine und besonders bevorzugt um eine sterile Streckblasmaschine.

Bei einem weiteren vorteilhaften Verfahren ist eine Beaufschlagungseinrichtung vorgesehen, welche mit wenigstens einem Teil des in den Behandlungsraum zurückzuführenden Mediums wenigstens einen Bereich der Kunststoffvorformlinge und insbesondere eine Außenoberfläche der Kunststoffvorformlinge beaufschlägt.

Bevorzugt wird dabei das zurückzuführende Medium mittels einem Gebläse beschleunigt. Bevorzugt weist die Beaufschlagungseinrichtung wenigstens eine und bevorzugt mehrere Düsen auf. Bevorzugt beaufschlagt die Beaufschlagungseinrichtung die Kunststoffbehältnisse und/oder Kunststoffvorformlinge und insbesondere die Kunststoffvorformlinge von unten her.

Weitere Vorteile und Ausführungsformen ergeben sich aus den beigefügten Zeichnungen:

### Dabei zeigen:

- Fig. 1a: eine schematische Darstellung einer erfindungsgemäßen Vorrichtung zur Innensterilisation;
- Fig. 1b: eine schematische Darstellung einer Vorrichtung zur Außensterilisation;
- Fig. 1c: eine schematische Darstellung einer Vorrichtung zur Innen- und Außensterilisation;
- Fig. 2: eine Darstellung zur Beaufschlagung von Kunststoffvorformlingen;
- Fig. 3: eine Darstellung zur Rückführung des Mediums; und
- Fig. 4: eine Darstellung zur Beaufschlagung der Außenoberfläche der Kunststoffvorformlinge.

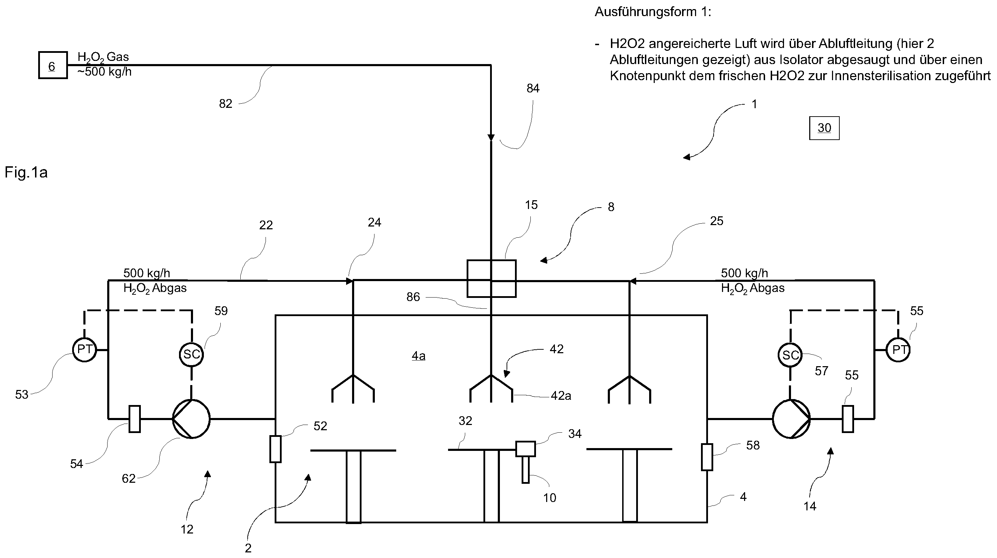

Fig. 1a zeigt eine erfindungsgemäße Vorrichtung 1 zum Behandeln von Kunststoffvorformlingen und/oder (Kunststoff)Behältnissen. Diese weist ein bevorzugt im Wesentlichen geschlossenes Gehäuse 4 auf, innerhalb dessen ein Behandlungsraum 4a zum Behandeln und insbesondere Sterilisieren, bevorzugt zur Innensterilisation von Kunststoffvorformlingen 10 ausgebildet ist.

Das Bezugszeichen 2 kennzeichnet in seiner Gesamtheit eine Transporteinrichtung, welche hier drei Transportsterne aufweist, die innerhalb des Behandlungsraums 4a nacheinander angeordnet sind, sodass jeder Kunststoffvorformling 10 von jeder dieser Transporteinheiten (nacheinander) transportiert wird.

Das Bezugszeichen 6 kennzeichnet eine Erzeugungseinrichtung, welche zum Erzeugen eines fließfähigen Sterilisationsmittels, insbesondere von H₂O₂ Gas geeignet und bestimmt ist. Besonders bevorzugt handelt es sich bei dieser Erzeugungseinrichtung um einen Verdampfer, welcher per se aus dem Stand der Technik bekannt ist.

Über eine Verbindungsleitung 82 und ein Ventil 84 sowie eine weitere Verbindungsleitung kann dieses Sterilisationsmedium dem Behandlungsraum 4a zugeführt werden. Bevorzugt handelt es sich bei dem Ventil 84 um ein steuerbares Ventil und insbesondere um ein pneumatisch oder elektrisch betätigtes Ventil.

Das Bezugszeichen 12 kennzeichnet eine erste Abführeinrichtung, welche aus dem Behandlungsraum 4a das darin befindliche Medium, d. h. insbesondere das mit Sterilisationsmittel angereicherte Gas abführen kann. Das Bezugszeichen 62 kennzeichnet eine Verdichtereinrichtung beispielsweise eine Pumpe zum Verdichten dieses aus dem Behandlungsraum entnommenen Sterilisationsmittels bzw. des Gases.

Das Bezugszeichen 22 kennzeichnet eine Leitungseinrichtung, welche das aus dem Behandlungsraum entnommene fließfähige Medium dem Behandlungsraum 4a zuführt. Das Bezugszeichen 24 kennzeichnet eine Ventileinrichtung zur Steuerung des in den Behandlungsraum zurückzuführenden gasförmigen Mediums. Bevorzugt handelt es sich auch bei der Ventileinrichtung 24 um ein steuerbares Ventil.

Das Bezugszeichen 14 kennzeichnet eine zweite Abführeinrichtung, die ebenfalls dazu geeignet und bestimmt ist, das Gas aus dem Behandlungsraum 4a abzuführen. Diese zweite Abführeinrichtung ist bevorzugt im Wesentlichen in der gleichen Weise gestaltet, wie die erste Abführeinrichtung 12. Das Bezugszeichen 25 kennzeichnet ein Ventil, welches die gleiche Funktion erfüllt wie die vorstehend beschriebene Ventileinrichtung 24, d.h. die Steuerung des dem Behandlungsraum 4a rückzuführenden fließfähigen Mediums.

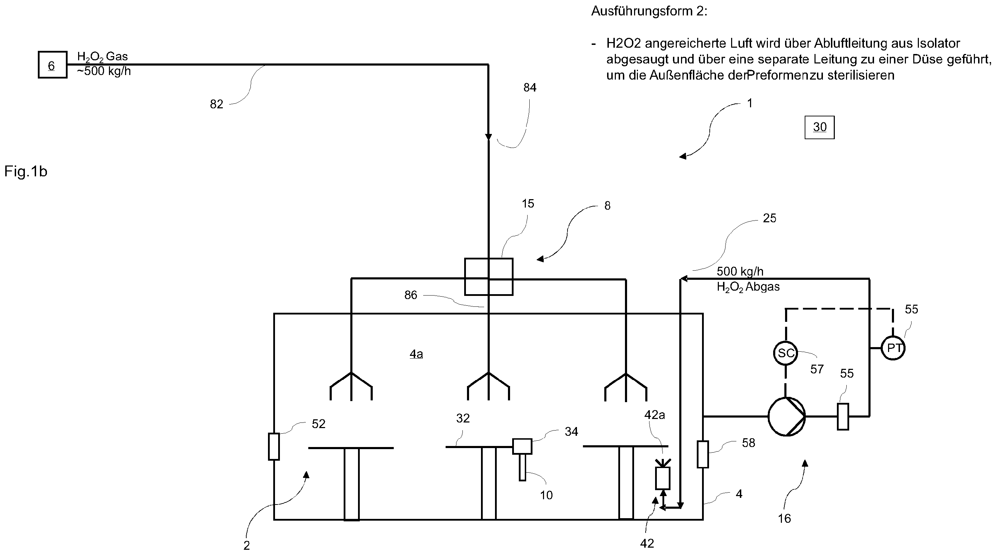

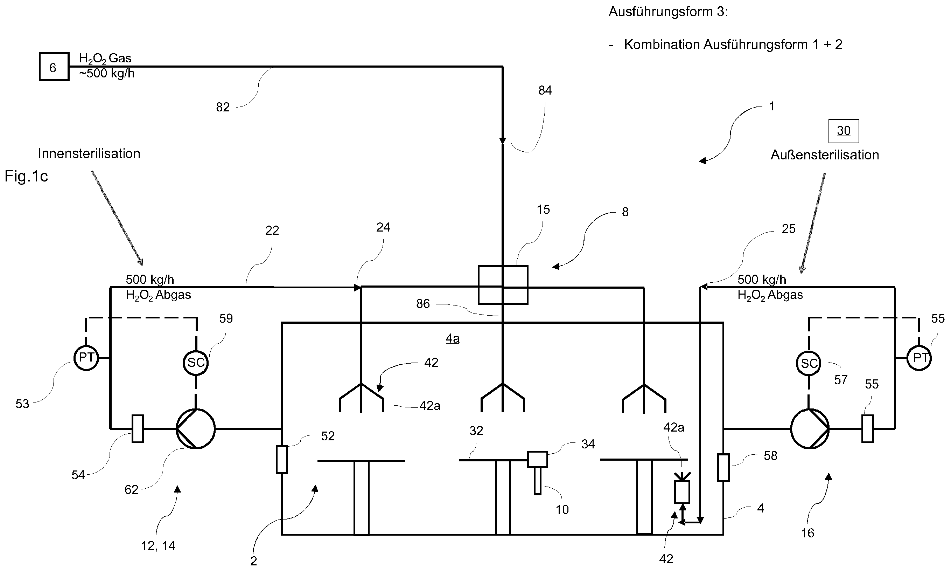

Das Bezugszeichen 15 kennzeichnet schematisch eine Mischeinrichtung, in welcher das entnommene Gas mit dem neu erzeugten Gas gemischt wird, um anschließend über eine Leitung 86 den Behandlungsraum 4a zugeführt zu werden. Weitere Mischeinrichtungen können auch in den weiteren in Fig. 1 gezeigten Knotenpunkten vorgesehen sein.

Die Transporteinrichtung 2 weist bevorzugt einen drehbaren Transportträger 32 auf, an dem besonders bevorzugt eine Vielzahl von Halteeinrichtungen 34 zum Halten von Kunststoffvorformlingen 10 angeordnet ist. Das Bezugszeichen 42 kennzeichnet eine Beaufschlagungseinrichtung, welche dazu dient, die Kunststoffvorformlinge 10 mit dem Sterilisationsmittel zu beaufschlagen. Dabei weist diese Beaufschlagungseinrichtung 42 eine Vielzahl von Beaufschlagungsdüsen 42a auf, welche jeweils das Sterilisationsmittel in die Kunststoffvorformlinge einbringen.

Bevorzugt, ist in dem Behandlungsraums eine Vielzahl von Sensoreinrichtungen 52,58 vorgesehen, die beispielsweise einen Gasdruck, einen Anteil an Sterilisationsmittel oder eine Temperatur erfassen können.

Weiterhin sind bevorzugt außerhalb des Behandlungsraums Sensoreinrichtungen 54,56, beispielsweise in Form von Durchflussmesseinrichtungen vorgesehen. Die Bezugszeichen 53, 55, 57, 59, stellen weitere Sensoreinrichtungen dar. Die Bezeichnung "SC" steht im gezeigten Beispiel für "Speed Control", wodurch verdeutlicht werden soll, dass beispielsweise eine Geschwindigkeit überwacht werden kann. Bei der überwachten Geschwindigkeit kann es sich beispielsweise um die Drehzahl der Pumpe handeln, die beispielsweise mittels eines Frequenzumformers gesteuert werden könnte.

Das Bezugszeichen 30 kennzeichnet eine Steuerungseinrichtung, welche zum Steuern und Regeln eines Mischungsverhältnisses des in den Behandlungsraum einzuführenden Gases ist. Bevorzugt werden dieser Steuerungseinrichtung 30 auch Messsignale und/oder Messwerte der einzelnen Sensoreinrichtungen zugeführt, und die Steuerung der Vorrichtung auch auf Basis dieser Messwerte durchgeführt.

Sowohl die schematisch links als auch rechts von dem im Wesentlichen geschlossenes Gehäuse 4, angeordneten Bereiche, mit dem Behandlungsraum 4a, sind zum Behandeln und insbesondere Sterilisieren, bevorzugt zur Innensterilisation von Kunststoffvorformlingen 10 vorgesehen und geeignet.

Es kann auch lediglich eine Abführeinrichtung wie 12,14 vorgesehen sein.

Fig. 1b zeigt eine ähnliche schematische Darstellung einer Vorrichtung 1 zum Behandeln von Kunststoffvorformlingen und/oder (Kunststoff)Behältnissen. Diese weist ein bevorzugt im Wesentlichen geschlossenes Gehäuse 4 auf, innerhalb dessen ein Behandlungsraum 4a zum Behandeln und insbesondere Sterilisieren, bevorzugt zur Außensterilisation von Kunststoffvorformlingen 10 ausgebildet ist.

Die Bezugszeichen mit zusätzlichen Merkmalen sind der Beschreibung zu Fig. 1a zu entnehmen. Mit gleichen Bezugszeichen versehene Elemente kennzeichnen entsprechende Elemente wie in Fig. 1a

Das Bezugszeichen 16 kennzeichnet eine Abführeinrichtung, die dazu geeignet und bestimmt ist, das Gas aus dem Behandlungsraum 4a abzuführen. Diese Abführeinrichtung 16 ist bevorzugt im Wesentlichen in der gleichen Weise gestaltet, wie die Abführeinrichtungen 12 und 14 in Fig. 1a.

Es kann auch darin ein zusätzliches Ventil, entsprechend dem Bezugszeichen 25 aus Fig. 1a vorgesehen sein.

Es wird dabei das Gas aus dem Behandlungsraum durch die Abführeinrichtung 16 in einer separaten Leitung, bevorzugt auch über ein Ventil oder einen Knotenpunkt mit frischem Gas (insbesondere angereichert mit Sterilisationsmittel) vermengt in den Behandlungsraum 4a geführt.

Daran schließt sich bevorzugt eine Beaufschlagungseinrichtung 42 an, welche dazu dient, die Kunststoffvorformlinge 10 mit dem Sterilisationsmittel zu beaufschlagen. Dabei weist diese Beaufschlagungseinrichtung 42 bevorzugt eine Vielzahl von Beaufschlagungsdüsen 42a, oder eine einzige Beaufschlagungsdüse 42a auf, welche jeweils das Sterilisationsmittel auf die Kunststoffvorformlinge aufbringen.

Es werden so bevorzugt die Außenflächen von Kunststoffvorformlingen sterilisiert.

Es können auch wenigstens zwei Abführeinrichtungen 16 vorgesehen sein.

Fig. 1c zeigt eine weitere ähnliche schematische Darstellung einer Vorrichtung 1 zum Behandeln von Kunststoffvorformlingen und/oder (Kunststoff)Behältnissen. Diese weist ein bevorzugt im Wesentlichen geschlossenes Gehäuse 4 auf, innerhalb dessen ein Behandlungsraum 4a zum Behandeln und insbesondere Sterilisieren, bevorzugt zur Innen- und Außensterilisation von Kunststoffvorformlingen 10 ausgebildet ist.

Es ist dabei schematisch links eine Abführeinrichtung 12,14 zur bevorzugten Innensterilisation, und schematisch rechts eine Abführeinrichtung 16 zur bevorzugten Außensterilisation von Kunststoffvorformlingen 10 ausgebildet.

Es können auch wenigstens zwei Abführeinrichtungen 12,14 zur bevorzugten Innensterilisation und wenigstens zwei Abführeinrichtungen 16, zur bevorzugten Außensterilisation, angebracht sein.

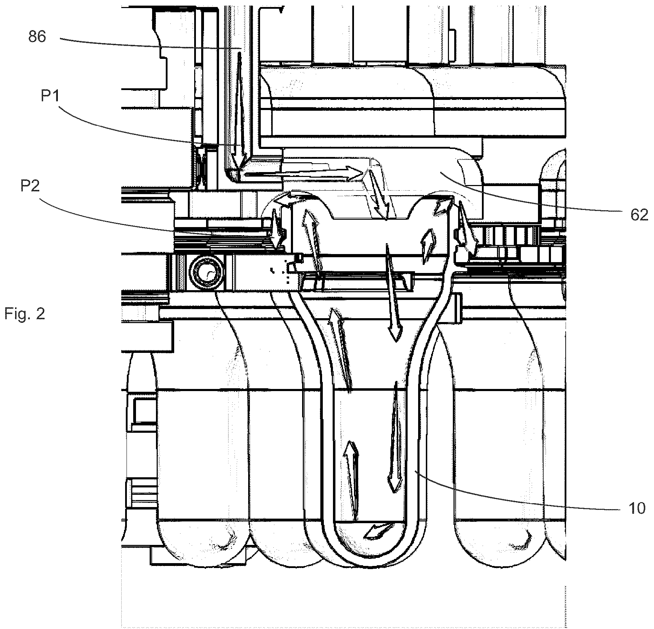

Fig. 2 zeigt eine detaillierte Darstellung zur Beaufschlagung eines Kunststoffvorformlings 10 genauer hier dessen Innenoberflächen. Über die Leitung 86 wird das in den Behandlungsraum (rück)geführte Medium in den Kunststoffvorformling eingeleitet (Pfeile P1). Oberhalb der Kunststoffvorformlinge befindet sich bevorzugt eine Verteileinrichtung 62, welche bewirkt, dass aus den Kunststoffvorformlingen wieder austretendes Medium auch an einen Außenbereich der Mündung der Kunststoffvorformlinge 10 gelangt.

Diese Verteileinrichtung 62 weist bevorzugt einen kreisförmigen Querschnitt auf.

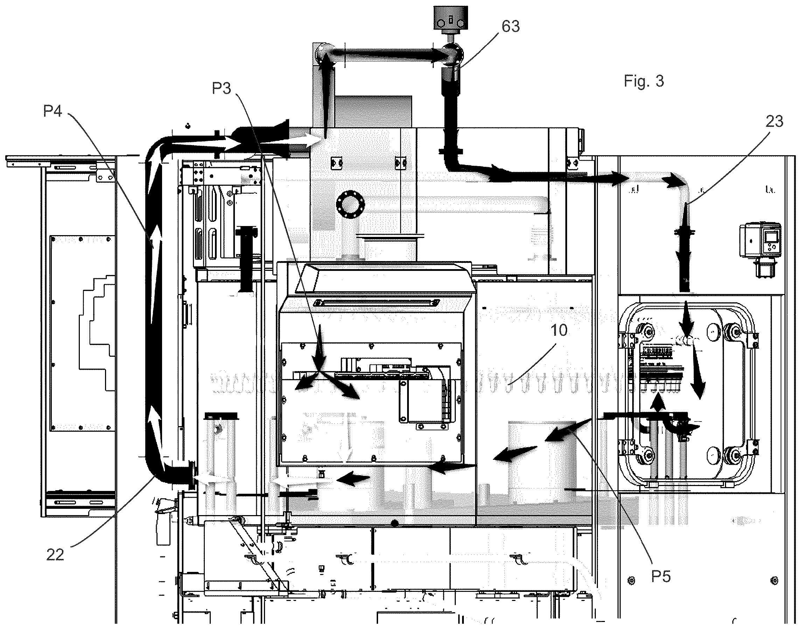

Fig. 3 zeigt eine Darstellung zur Veranschaulichung der Rückführung des dem Behandlungsraum entnommenen Mediums und dessen Verwendung zum Sterilisieren der Außenoberflächen.

Über eine Entnahmeleitung 22 wird das Medium aus dem Behandlungsraum ausgeführt. Diese Entnahmeleitung ist dabei bevorzugt in einem unteren Bereich des Behandlungsraums angeordnet. Das Medium strömt dann (Pfeil P4 und P3) zu einer Verdichtereinrichtung 63 und gelangt über eine Leitung wieder in den Behandlungsraum (Pfeil P5).

Die Pfeile P3 veranschaulichen die Beaufschlagung der jeweiligen Innenräume der Kunststoffvorformlinge.

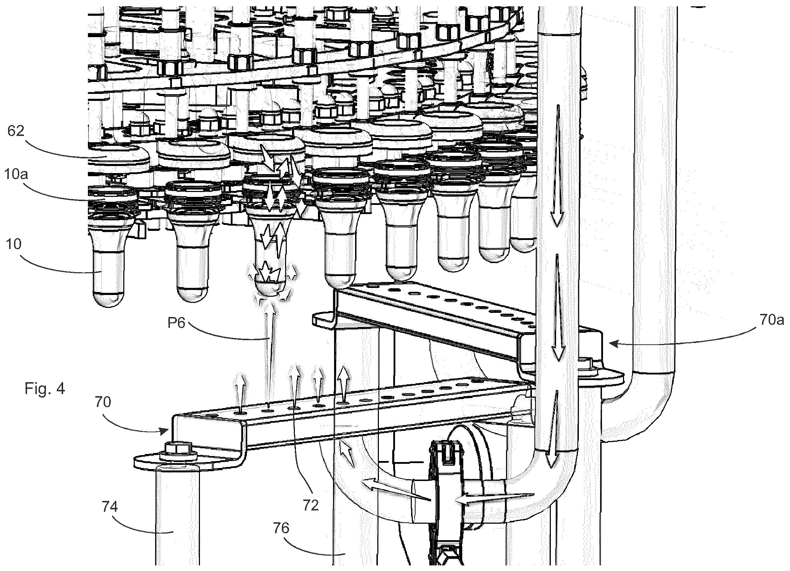

Fig. 4 zeigt die Außenbeaufschlagung der einzelnen Kunststoffvorformlinge. Innerhalb des Behandlungsraums ist hier wenigstens eine Beaufschlagungsanordnung 70, 70a vorgesehen. Diese Beaufschlagungsanordnung weist hier eine Vielzahl von Düsen 72 auf, die hier bevorzugt einreihig hintereinander angeordnet sind.

Diese Düsen beaufschlagen bevorzugt die Kunststoffvorformlinge 10 von unten her.

Die Bezugszeichen 74 und 76 kennzeichnen Träger zum Tragen der beiden Beaufschlagungsanordnungen 70,70a. Die Beaufschlagungsanordnungen sind bevorzugt in der vertikalen Richtung verstellbar, um so eine Anpassung an unterschiedliche Geometrien der Kunststoffvorformlinge zu ermöglichen.

Es wird darauf hingewiesen, dass sämtliche Merkmale, welche unter Bezugnahme auf das Verfahren beschrieben wurden, in entsprechender Weise auch für die Vorrichtung offenbart sind, was insbesondere bedeutet, dass die entsprechende Vorrichtung Einrichtungen aufweist, welche zur Durchführung der jeweiligen Verfahren geeignet und bestimmt sind. Weiterhin sind auch Merkmale, welche unter Bezugnahme auf die Vorrichtung beschrieben wurden entsprechend für das oder die Verfahren anwendbar. Dies bedeutet, dass die Verfahren unter Verwendung der entsprechenden Vorrichtungsmerkmale ausgeführt werden.

Die Anmelderin behält sich vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung zum Behandeln und insbesondere Sterilisieren von Kunststoffvorformlingen und/oder Behältnissen mit einer Transporteinrichtung (2), welche dazu geeignet und bestimmt ist, die Kunststoffvorformlinge und/oder Behältnisse während ihrer Behandlung zu transportieren, mit einem einen Behandlungsraum (4a) ausbildenden Gehäuse (4), innerhalb dessen die Transporteinrichtung (2) angeordnet ist, mit einer Erzeugungseinrichtung (6) zum Erzeugen eines fließfähigen Behandlungsmittels und insbesondere Sterilisationsmittels und mit einer Zuführeinrichtung (8) welche dazu geeignet und bestimmt ist, das von der Erzeugungseinrichtung erzeugte Sterilisationsmittel dem Behandlungsraum zuzuführen,
**dadurch gekennzeichnet, dass**
die Vorrichtung eine erste Abführeinrichtung (12) aufweist, welche dazu geeignet und bestimmt ist, aus dem Behandlungsraum ein mit dem Sterilisationsmittel angereichertes fließfähiges Medium zu entnehmen sowie eine Rückführungseinrichtung (22, 24, 26), welche dazu geeignet und bestimmt ist, wenigstens einen Teil des aus dem Behandlungsraum entnommenen Mediums wieder in diesen Behandlungsraum und/oder einen weiteren Behandlungsraum zurückzuführen.

2. Vorrichtung (1) nach Anspruch 1,
**dadurch gekennzeichnet, dass**
die Vorrichtung wenigstens eine Mischeinrichtung aufweist, um wenigstens einen Teil des aus dem Behandlungsraum entnommenen fließfähigen Mediums mit dem von der Erzeugungseinrichtung erzeugten fließfähigen Medium zu mischen.

3. Vorrichtung (1) nach wenigstens einem der vorangegangenen Ansprüche,
**dadurch gekennzeichnet, dass**
die Vorrichtung (1) eine Steuerungseinrichtung aufweist, welche dazu geeignet und bestimmt ist, ein Mischungsverhältnis des dem Behandlungsraum zuzuführenden und insbesondere auf die Anteile des von der Erzeugungseinrichtung erzeugten Mediums und des von dem Behandlungsraum entnommenen Mediums zu steuern.

4. Vorrichtung (1) nach wenigstens einem der vorangegangenen Ansprüche,
**dadurch gekennzeichnet, dass**
die Vorrichtung eine zweite Abführeinrichtung (14) aufweist, welche dazu geeignet und bestimmt ist, aus dem Behandlungsraum ein mit dem Sterilisationsmittel angereichertes fließfähiges Medium zu entnehmen, wobei bevorzugt diese zweite Abführeinrichtung (14) von der ersten Abführeinrichtung (12) beanstandet ist.

5. Vorrichtung (1) nach wenigstens einem der vorangegangenen Ansprüche,
**dadurch gekennzeichnet, dass**
die Vorrichtung (1) wenigstens eine innerhalb des Behandlungsraums angeordnete Beaufschlagungseinrichtung (42) aufweist, um die von der Transporteinrichtung transportierten Kunststoffvorformlinge oder Behältnisse mit dem Sterilisationsmittel zu beaufschlagen.

6. Vorrichtung (1) nach wenigstens einem der vorangegangenen Ansprüche,
**dadurch gekennzeichnet, dass**
die Vorrichtung (1) wenigstens eine Sensoreinrichtung (52, 54, 56) aufweist, welche dazu geeignet und bestimmt ist, wenigstens einen Messwert zu erfassen, der für das innerhalb des Behandlungsraums befindliche fließfähige Medium charakteristisch ist, wobei bevorzugt dieser Messwert aus einer Gruppe von Messwerten ausgewählt ist, welche Druckmesswerte, Temperaturmesswerte, Messwerte für einen Anteils eines Sterilisationsmittels, Messwerte für eine Konzentration des Sterilisationsmittels, Luftfeuchtigkeitsmesswerte und dergleichen enthält.

7. Vorrichtung (1) nach wenigstens einem der vorangegangenen Ansprüche,
**dadurch gekennzeichnet, dass**
die Vorrichtung wenigstens eine Durchflussmesseinrichtung aufweist.

8. Vorrichtung (1) nach wenigstens einem der vorangegangenen Ansprüche,
**dadurch gekennzeichnet, dass**
die Vorrichtung eine Verdichtereinrichtung zur Verdichtung des von der Erzeugungseinrichtung erzeugten fließfähigen Mediums und/oder des aus dem Behandlungsraum entnommenen fließfähigen Mediums aufweist.

9. Vorrichtung (1) nach wenigstens einem der vorangegangenen Ansprüche,
**dadurch gekennzeichnet, dass**
die Vorrichtung (1) eine Beaufschlagungseinrichtung (72) aufweist, welche dazu geeignet und bestimmt ist, mit wenigstens einem Teil des in den Behandlungsraum zurückzuführenden Mediums wenigstens einen Bereich der Kunststoffvorformlinge (10) oder Kunststoffbehältnisse und insbesondere eine Außenoberfläche der Kunststoffvorformlinge (10) oder Kunststoffbehältnisse zu beaufschlagen.

10. Verfahren zum Behandeln und insbesondere Sterilisieren von Kunststoffvorformlingen und/oder Behältnissen mit einer Transporteinrichtung (2), welche die Kunststoffvorformlinge und/oder Behältnisse während ihrer Behandlung wenigstens zeitweise transportiert, mit einem einen Behandlungsraum (4a) ausbildenden Gehäuse (4), innerhalb dessen die Transporteinrichtung (2) angeordnet ist, mit einer Erzeugungseinrichtung (6) welche ein fließfähiges Behandlungsmittel und insbesondere Sterilisationsmittel erzeugt und mit einer Zuführeinrichtung (8) welche das von der Erzeugungseinrichtung erzeugte Sterilisationsmittel dem Reinraum zuführt,
**dadurch gekennzeichnet, dass**
eine erste Abführeinrichtung (12) vorgesehen ist, welche aus dem Behandlungsraum ein mit dem Sterilisationsmittel angereichertes fließfähiges Medium entnimmt sowie eine Rückführungseinrichtung (22, 24, 26), welche wenigstens einen Teil des aus dem Behandlungsraum entnommenen Mediums wieder in diesen Behandlungsraum zurückzuführt.

11. Verfahren nach dem vorangegangenen Anspruch,
**dadurch gekennzeichnet, dass**
die Kunststoffvorformlinge und/oder Behältnisse innerhalb des Behandlungsraums wenigstens abschnittsweise auf einem kreisförmigen Transportpfad transportiert werden und/oder die Kunststoffvorformlinge und/oder Behältnisse wenigstens einmal innerhalb des Behandlungsraums von einer ersten Halteeinrichtung an eine zweite Halteeinrichtung übergeben werden.

12. Verfahren nach wenigstens einem der vorangegangenen Ansprüche,
**dadurch gekennzeichnet, dass**
wenigstens ein Teil des aus dem Behandlungsraum entnommenen fließfähigen Mediums mit dem von der Erzeugungseinrichtung erzeugten fließfähigen Medium gemischt wird, wobei dieses Mischen bevorzugt außerhalb des Behandlungsraums erfolgt.

13. Verfahren nach wenigstens einem der vorangegangenen Ansprüche,
**dadurch gekennzeichnet, dass**
ein Mischungsverhältnis zwischen dem von der Erzeugungseinrichtung erzeugten Mediums und dem von dem Behandlungsraum entnommenen Mediums gesteuert und/oder geregelt wird.

14. Verfahren nach wenigstens einem der vorangegangenen Ansprüche,
**dadurch gekennzeichnet, dass**
die Kunststoffvorformlinge und/oder Behältnisse dem Behandlungsraum mittels einer Schleuseneinrichtung zugeführt werden und/oder die Kunststoffvorformlinge und/oder Behältnisse dem Behandlungsraum mittels einer Schleuseneinrichtung abgeführt werden und/oder die Kunststoffvorformlinge und/oder Behältnisse erwärmt werden, bevor sie dem Behandlungsraum zugeführt werden.

15. Verfahren nach wenigstens einem der vorangegangenen Ansprüche,
**dadurch gekennzeichnet, dass**
eine Beaufschlagungseinrichtung vorgesehen ist, welche mit wenigstens einem Teil des in den Behandlungsraum zurückzuführenden Medium wenigstens einen Bereich der Kunststoffvorformlinge und insbesondere eine Außenoberfläche der Kunststoffvorformlinge beaufschlagt.
